(19) Europäisches Patentamt

European Patent Office

Office européen des brevets

(11) **EP 1 719 533 A1**

(12) **EUROPEAN PATENT APPLICATION**

(43) Date of publication:
**08.11.2006 Bulletin 2006/45**

(51) Int Cl.:
*A61L 27/36* (2006.01)    *A61L 31/14* (2006.01)

(21) Application number: **05009748.4**

(22) Date of filing: **04.05.2005**

(84) Designated Contracting States:
**AT BE BG CH CY CZ DE DK EE ES FI FR GB GR
HU IE IS IT LI LT LU MC NL PL PT RO SE SI SK TR**
Designated Extension States:
**AL BA HR LV MK YU**

(71) Applicant: **Tecnobiomedica S.p.A.
00040 Pomezia (RM) (IT)**

(72) Inventors:
• **Piconi, Corrado
00040 Pomezia
(Roma) (IT)**

• **Rinaldi, Stefano
00040 Pomezia
(Roma) (IT)**
• **Della Ciana, Leopoldo
00040 Pomezia
(Roma) (IT)**
• **Motta, Antonella
00040 Pomezia
(Roma) (IT)**

(74) Representative: **Freyria Fava, Cristina
Buzzi, Notaro & Antonielli d'Oulx Srl,
Via Maria Vittoria 18
10123 Torino (IT)**

(54) **Method of preparing acellularized, biocompatible, implantable material**

(57)    The present invention relates to new method of preparing acellularized, biocompatible, implantation material starting from a biological material. In particular, the present invention relates to a method of preparing biological material for implantation comprising (a) fixation with an aldehyde the biological material and (b) detoxification/anticalcification of the fixed biological material, wherein the detoxification/anticalcification step makes use of a novel class of substances of formula:

$$NH_2\text{-}(CH_2)_n\text{-}X \qquad (I)$$

wherein
X is COOH, SO_3H, PO_3H_2 and
n is an integer from 2 to 6.

EP 1 719 533 A1

Printed by Jouve, 75001 PARIS (FR)

**Description**

The present invention relates to a multistep, synergistic method of preparing acellularized, biocompatible implantation material starting from a biological material.

Biological materials of various origin to replace diseased or malfunctioning tissues have been in use for more than forty years. Such materials can be obtained from the patient (autologous tissue), from other individuals belonging to the same species (homologous tissue) or from other species (heterologous tissue). The source of the materials can be heart valves, pericardium, tendons, arteries, veins, dura mater, ligaments and bones.

Since all tissues not obtained from the patient are subject to rejection, it was found necessary, from the very beginning, to develop chemical treatments capable of reducing the antigenic properties of these tissues and at the same time improving their mechanical properties and resistance to enzymatic degradation.

The most commonly used substance for the purpose of fixing biological tissues is glutaraldehyde. Its success is most certainly due to its high crosslinking capability and to its mix of hydrophilic and hydrophobic properties, which allow the molecule to rapidly diffuse into the matrix. It can also mask antigenic determinants and thus suppress the immunological recognition of the tissues. Glutaraldehyde is also a powerful sterilizing agent. No other single compound exhibits such a range of useful properties.

Nevertheless, the use of glutaraldehyde to fix biological tissues does induce several adverse effects, such as cytotoxicity and mineralization. Many authors have considered the presence of unreacted aldehyde groups as the main cause of cytotoxicity and of the development of inflammatory reactions. Aldehyde groups are also one of the contributory causes of tissue calcification.

In order to detoxify biological materials treated with glutaraldehyde, molecules of various kind have been used. These detoxification methods generally rely on the formation of Schiff bases or imino groups between residual aldehyde groups and amino-containing substances. Thus, Girardot, M.N., et al., "Alpha-Aminoleic Acid, A New Compound,Prevents Calcification of Bioprostheticheart Valves", The 17th Annual Meeting of the Society for Biomaterials, May 1-5, 1991, p. 1141, describes the use of 2-aminooleic acid for this purpose.

Other detoxification/anticalcification approaches include the use of chondroitin sulfate, protamine, aminodifosfonates and chitosans.

Especially effective methods for detoxifying biological materials treated with glutaraldehyde are based on use of aminodicarboxylic acids, e.g. glutamic acid, as disclosed in the US patent No. 5 188 834 and aminocarboxylic acid, e.g. homocysteic acid, as disclosed in US-A-5 873 812.

The object of the present invention is to provide an alternative and more efficient method of preparing acellularized, biocompatible implantable materials.

According to the present invention, that object is achieved thanks to a method having the characteristics referred to specifically in the ensuing claims. In particular, the object of the present invention is achieved by the use of a novel class of substances for the detoxification/anticalcification of biological tissues. These substances are, in fact, more reactive than the known substances used in the past and are more effective in penetrating biological structures and reduce disruption of the tissues.

The claims form an integral part of the disclosure of the invention provided herein.

The invention will now be described, purely by way of non-limiting example, with reference to the annexed figures, in which:

- Figure 1 is a SEM image showing the pericardium after mechanical cleaning (step A);
- Figure 2 shows a picture taken with ESEM of pericardium after treatment with glutaraldehyde followed by 72 hours of storage in paraben;
- Figure 3 is a graph showing data obtained with the LDH test on bovine pericardium seeded with human fibroblasts from embryo pulmonary tissue (MRC5 line);
- Figure 4 is a graph showing the results of Neutral Red Assay on bovine pericardium seeded with human fibroblasts from embryo pulmonary tissue (MRC5 line);
- Figure 5 is a graph showing measurement of alkaline phosphatase (ALP) activity on bovine pericardium seeded with human fibroblasts from embryo pulmonary tissue (MRC5 line);
- Figure 6 is a SEM image of bovine pericardium after treatment with the method of the present invention seeded with human fibroblasts from embryo pulmonary tissue (MRC5 line) taken 72 hours after seeding.

The present invention makes use of a novel class of substances for the detoxification/anticalcification of biological tissues. It was found that compounds having the general formula:

$$NH_2\text{-}(CH_2)_n\text{-}X \qquad (I)$$

wherein

X is COOH, SO$_3$H or PO$_3$H$_2$ and

n is an integer from 2 to 6

are very effective for neutralizing excess aldehyde groups and at same time in preventing tissue calcification.

Compounds represented by the general formula (I) - useful according to the present invention - are:

> 3-aminopropanoic acid also known as β-alanine;
> 4-aminobutanoic also known as γ-aminobutyric acid (GABA);
> 5-aminopentanoic acid;
> 6-aminohexanoic acid;
> 2-aminoethanesulfonic acid, also known as taurine;
> 3-aminopropane-1-sulfonic acid;
> 4-aminobutane-1-sulfonic acid;
> 5-aminopentane-1-sulfonic acid;
> 6-aminohexane-1-sulfonic acid;
> 2-aminoethanephosphonic acid;
> 3-aminopropane-1-phosphonic acid;
> 4-aminobutane-1-phosphonic acid;
> 5-aminopentane-1-phosphonic acid;
> 6-aminohexane-1-phosphonic acid.

Among these compounds, especially effective were found γ-aminobutyric acid (GABA) and 3-aminopropane-1-sulfonic acid.

The main difference between such compounds and those described in US-A-5 188 834 and US-A-5 873 812 is the absence of the carboxyl group bound to the same carbon atom as the amino functionality. The basicity and nucleofilicity and hence the reactivity of primary amines in α-aminoacids are reduced by the inductive effect caused by the carboxyl group in close proximity. Thus, the pK$_5$ of glycine is 9.60, compared to 10.65 of a primary amine, such as metilamine; pK$_2$ for glutamic acid is 9.76 vs. 10.56 for γ-aminobutyric.

Moreover, compounds described by the general formula (I) have not net charge in a wide pH interval, and especially at physiological pH, while α-aminoacids with acidic side chains as those previously described, and in particular glutamic acid and homocysteic acid have an excess of negative charge at pH values higher than about pH 4.2 and 2.2, respectively. Neutral species are more effective in penetrating biological structures and reduce disruption of the tissues.

Additionally, compounds such as γ-aminobutyric are much more water soluble than glutamic acid and, therefore, the solution prepared with it are more concentrated and thus more effective than those containing glutamic acid as detoxifier/anticalcifier.

Treatment with the compounds described by formula (I) is carried out with acidic aqueous solution having pH within the range from 3.0 to 5.0 and, preferably, between 3.0 and 3.5. The reaction medium is a buffered solution, preferably belonging to the group of sodium citrate/HCl, potassium hydrogen phthalate/HCl, citric acid/phosphate, and citrate-phosphate-borate/HCL. The concentration of the acid solutions is generally within the range from 10 mmM to saturated solutions, more preferably from 25 to 100 mM.

In order to fully exploit the advantages of the novel reagents, it was found useful to develop a complete protocol, including steps before the fixation with glutaraldehyde and post-treatment steps after the detoxification/anticalcification procedure. More specifically, according to the present invention the biological material is subjected to a carefully designed sequence of treatments, namely cleaning of the sample, osmotic shock, extraction of cellular debris with detergents, fixation with an aldehyde, detoxification/anticalcification with an α,ω aminoacid, reconditioning and storage.

Thus, a complete treatment of biological material for implantation consists of the following steps, or procedures: (a) thorough mechanical cleaning of the tissue; (b) osmotic shock; (c) decellularization; (d) fixation; (e) detoxification/anticalcification; (f) reconditioning and storage in buffer.

Step (a) requires careful removal of connective and fat tissue, while keeping the sample at 0°C.

Next, the tissue undergoes one or more treatments aimed at removing the cellular components. As is known to the art, it may be preferable to first subject the tissue to osmotic shock (b), by alternatively treating it with distilled water and saline buffers. This procedure destroys the integrity of cellular walls and removes part of the cellular debris.

The remaining of this debris is more thoroughly removed by employing washing steps with detergents (c). Again, it is well known to the art the use of ionic detergents, such as sodium dodecyl solfate (SAS), nonionic detergents, such as Treiton X-100, zwitterionic detergents, sodium deoxycholate. Mixtures of the aforesaid detergents are also often used, to increase their effectiveness. Additionally, enzymes such as DNAses and RNAses, as well as common chelators, such as EDTA are added to the detergent buffer. Certain solvent of medium polarity can also be used, alone or together with detergent to remove said cellular debris, especially acetone, lower alcohols, chlorinated hydrocarbons, such as dichloromethane, chloroform, alone or in mixtures.

After rinsing off the excess detergent with a buffer solution, the sample is subjected to the fixation procedure (d), which is generally accomplished by immersing the sample in a dilute solution of glutaraldehyde, for several hours, up to a few days.

The fixation is followed by detoxification/anticalcification (e) with the compounds of formula (I), and especially with γ-aminobutyric acid (GABA) and 3-aminopropane-1-sulfonic acid.

It is advantageous to first treat the fixed tissues with a pH 3.3 citrate buffer, to help depolymerisation of glutaraldehyde oligomers formed under the experimental condition. These polymerisation reactions are, in fact, known to be reversible at lower pH values. As a results, masked aldehyde groups become exposed and can react with the amino groups of detoxifying agent in the next step. If prewashing with acidic buffer is not performed at this point, aldehyde groups masked by the aldolic self-condensation of glutaraldehyde would be i) slowly released spontaneously and ii) eventually responsible for a delayed increase in cytotoxicity of the implanted tissue.

For similar reasons, the detoxification/anticalcification step with γ-aminobutyric acid (GABA) and 3-aminopropane-1-sulfonic acid, or other compounds described by the general formula (I), is performed at acidic pH and, in particular, at pH 3.3.

The final step (f) requires reconditioning the sample at neutral pH and storage in a biologically compatible sterilizing solution, such as a paraben solution (0.02% n-propyl p-hydroxybenzoate and 0.18% methyl p-hydroxybenzoate).

The following example illustrates the invention in a detailed manner.

EXAMPLE

STEP A. Immediately after the explant, bovine pericardium from the local abattoir was placed in sterile physiological solution (0.9% NaCl) kept at 4°C.

A sample (12 x 15 cm) was carefully cleaned by removal of connective tissue and fat residues, while working at 0°C.

STEP B. Subsequently, the sample was subjected to osmotic shock, by washing it alternatively with distilled water and saline solution (with ice) at least five times. It is important to thoroughly rinse the sample and then store it for at least 20 minutes in distilled water, before the next immersion in saline solution. The purpose of this treatment is to break cellular walls and then remove cellular components as much as possible.

STEP C. The sample is then washed with a detergent solution formulated as follows: 1% Triton X-100, 0.25 M sodium deoxycholate, 0.02% EDTA, 0.1% DNAase and RNAase under continuous stirring for 48 hours at 37°C. After the first 24 hours the detergent solution was replaced with fresh solution. The sample was washed thoroughly (at least 20 minutes) with phosphate saline buffer (PBS) three times.

STEP D. The sample was then fixed with 0.5% glutaraldehyde in pH 7.4 PBS buffer at 0°C for 24 hours. At the end of this period the sample was subjected to two, 20 minutes washes with a pH 3.3, 0.16 M citrate buffer to remove glutaraldehyde in excess.

STEP E. A detoxification/anticalcification step was performed by treating the sample, for a total of three times, with a 60 mM solution of γ-aminobutyric acid (GABA) in pH 3.3, 0.1 M citrate buffer. This first treatment lasted 24 hr, the second 36 hours, and the third, 24 hours. The pH of the solution was checked, and if necessary, adjusted to pH 3.3 with HCl or NaOH.

STEP F. The final step, was performed by reconditioning the sample by rinsing three times for 20 minutes with saline solution (0.9 % NaCl in water). It is important that the sample does not contain the aminoacid employed in the detoxification/anticalcification step. It was stored in a paraben solution (0.02% n-propyl p-hydroxybenzoate and 0.18% methyl p-hydroxybenzoate).

After each step the sample was observed by electron microscopy (SEM and ESEM).

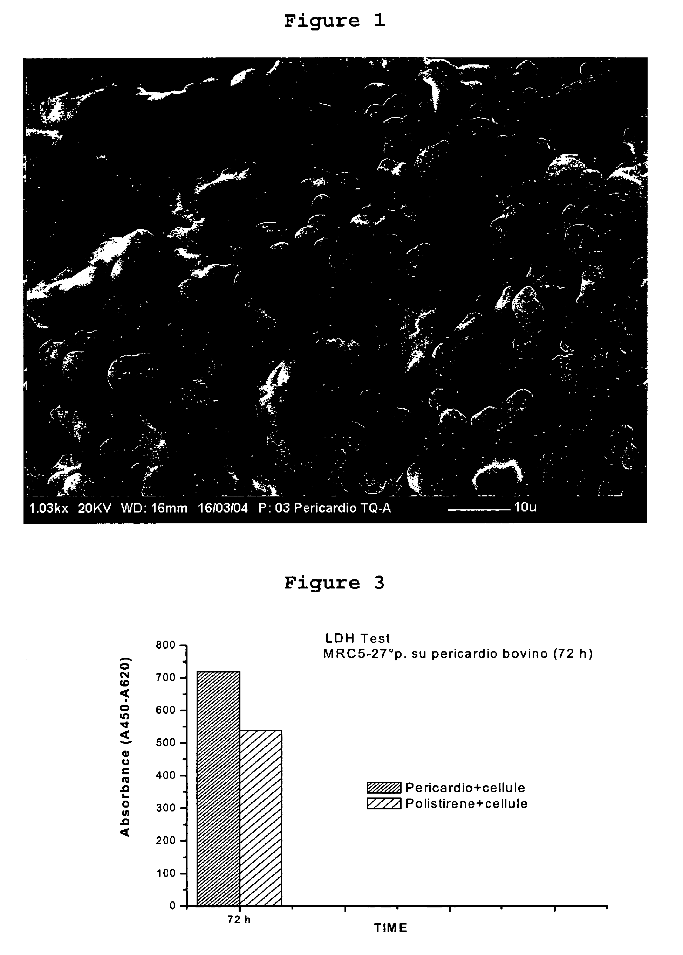

Figure 1 (SEM) shows the sample after step A, mechanical cleaning. The sample surface shows the presence of cellular components. Collagen fibers on the surface are well covered with fat layers.

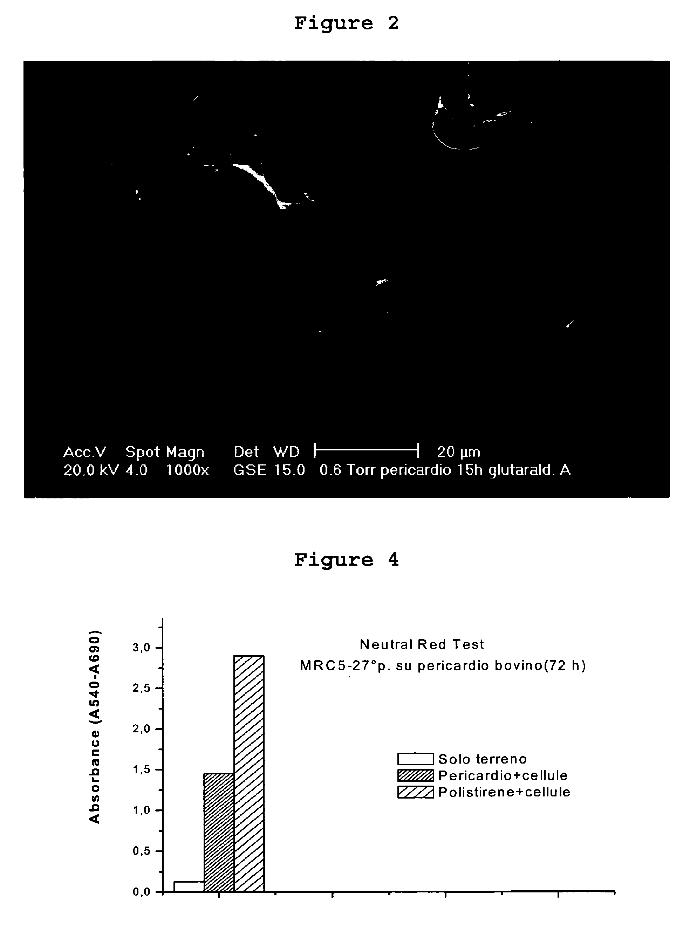

Figure 2 shows pictures taken with ESEM, in Low-Vacuum mode, after 72 hours of storage in paraben. The collagen structure appears to have been modified by the glutaraldehyde treatment but anatomically within the norm.

Cytotoxicity and cytolysis were evaluated by lactate dehydrogenase (LDH) and neutral red assays.

LDH is a stable cytoplasmatic enzyme found in most cells. It is released when the cytoplasmatic membrane is damaged. The assay is based on the conversion of yellow tetetrazolium salts into a red formazan dye (absorbance maximum at 500 nm). By measuring the activity of LDH released by damaged or dead cells it is possible to evaluate any cytotoxic or cytolytic effect due to the material onto which test cell are grown. Any increase in the number of damaged/dead cell is followed by a correspondent increase in LDH activity and is directly proportional to the amount of formazan produced. In a quantitative form, it can be stated as follows (LDH Cytotoxicity Detection Kit Manual, TAKARA Cat. MK 401):

$$Cytotoxicity\% = \left[ \left( espectedvalue - control(low) \right) / \left( control(high) - control(low) \right) \right] \times 100$$

(II)

Neutral red assay is a way of measuring the number of vital cells by absorption of the dye into the cells. Live cells absorb this dye and incorporate it into their liposomes. An increase/decrease in the number of cells and their physiological well-being correspond to an increase/decrease in the amount of neutral red incorporated by culture cells. The method was implemented by means of a SIGMA kit (No. TOX-4). Best results are obtained when cell are in logarithmic growth and at concentration less or equal to $10^6$ cell/ml.

A sample of bovine pericardium, treated as in the example, was seeded with human fibroblasts from embryo pulmonary tissue (MRC5 line). Data obtained with the LDH test, reported in Figure 3, show complete lack of cytotoxicity. The control is polystyrene (empty well). Expected value for pericardium (72 hours after seeding) is 1.66 $\pm$ 0.36. Expected value for control is 1.98 $\pm$ 0.20. Figure 4 shows the results of neutral red assay. Cells seeded on pericardium after 72 hours are 1.66E+05, cells seeded on control 72 hours after seeding are 3.06+05.

Since, in the absence of cytotoxicity, a decrease in the tendency to proliferate arises in the presence of an increased cellular activity, the samples were also tested for cellular activity.

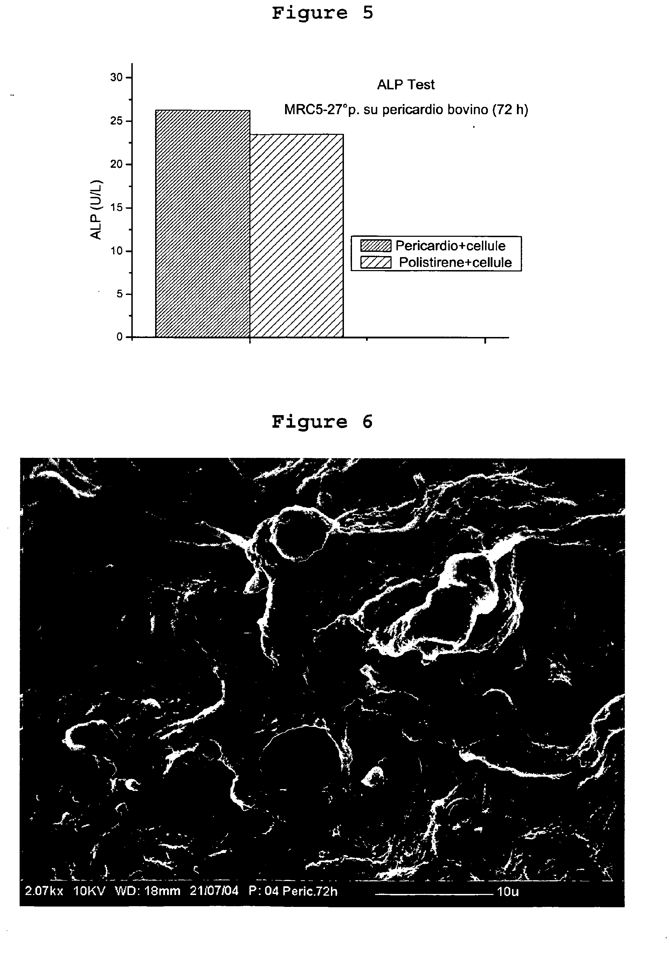

A test based on the quantitative measurement of alkaline phosphatase (ALP) was used. This enzyme, widely distributed in tissues, hydrolyses p-nitrophenol phosphate (colorless) to p-nitrophenol red at basic pH. The absorbance maximum of p-nitrophenol is at 405 nm. Therefore the absorbance increase at 405 nm is directly proportional to ALP activity. A Sigma kit (No. 245) was used for this test. Results are reported in Figure 5. Cellular activity is twice as high for cells seeded on pericardium than for those seeded on polystyrene (control), thus compensating the decrease in proliferation.

These results are consistent with SEM images taken 72 hours after seeding with MRC5 cells, Figure 6. Good adhesion of the seeded cells to pericardium substrate is evident in the picture, numerous cells are adherent to the substrate and cell interconnections can also be observed.

Those of skill in the art will promptly appreciate that all the numerical values provided herein are to be understood by taking into account the tolerances currently associated with determining/measuring such values.

Of course, without prejudice to the principle of the invention, the details of fabrication and the embodiments may vary widely with respect to what is described and illustrated herein, without thereby departing from the scope of the present invention, as defined by the annexed claims.

## Claims

1.   Method of preparing a biocompatible implantable material comprising (a) fixation of a biological implantable material with an aldehyde and (b) treating the fixed biological implantable material with an aminoacid in a reaction medium, **characterized in that** the aminoacid has the formula (I)

$$NH_2 - (CH_2)_n - X \qquad (I)$$

wherein
X is COOH, $SO_3H$, $PO_3H_2$ and
n is an integer from 2 to 6.

2.   Method according to claim 1, **characterized in that** the aminoacid is selected from the group consisting of $\gamma$-aminobutyric acid and 3-aminopropane-1-sulfonic acid.

3.   Method according to any one preceding claims, **characterized in that** the reaction medium is an acidic aqueous solution having pH within the range from about 3.0 to about 5.0.

4.   Method according to any one preceding claims, **characterized in that** the reaction medium is an acidic aqueous solution having pH within the range from about 3.0 to about 3.5.

5.   Method according to any one preceding claims, **characterized in that** the reaction medium is a buffered solution selected from the group consisting of sodium citrate/HCl, potassium hydrogen phthalate/HCl, citric acid/phosphate, and citrate-phosphate-borate/HCL.

6. Method according to any one preceding claims, **characterized in that** the concentration of the aminoacid in the reaction medium is within the range from about 10 mM to saturation.

7. Method according to any one preceding claims, **characterized in that** the concentration of the aminoacid in the reaction medium is within the range from about 25 mM to about 100 mM.

8. Method according to any one preceding claims, **characterized in that** before step (b) the fixed biological implantable material is treated with a citric buffer solution having pH about 3.3.

9. Method according to any one preceding claims, **characterized in that** before step (a) the biological implantable material is subjected to mechanical cleaning.

10. Method according to claim 9, **characterized in that** after mechanical cleaning the biological implantable material is subjected to an osmotic shock.

11. Method according to claim 10, **characterized in that** after osmotic shock the biological implantable material is subjected to a decellularization process.

12. Method according to any one of the preceding claims, **characterized in that** the aldehyde used in step (a) is glutaraldehyde.

13. Method according to any one of the preceding claims, **characterized in that** after step (b) the biological implantable material is subjected to a reconditioning step.

14. Use of an aminoacid of formula (I)

$$NH_2\text{-}(CH_2)_n\text{-}X \qquad (I)$$

wherein
X is COOH, $SO_3H$, $PO_3H_2$ and
n is an integer from 2 to 6
in a reaction medium for treating a biological implantable material for preparing a biocompatible implantable material.

15. Use according to claim 14, **characterized in that** the aminoacid is selected from the group consisting of γ-aminobutyric acid and 3-aminopropanesulfonic acid.

16. Use according to claim 14 or claim 15, **characterized in that** the reaction medium is an acidic aqueous solution having pH within the range from about 3.0 to about 5.0.

17. Use according to claim 14 or claim 15, **characterized in that** the reaction medium is an acidic aqueous solution having pH within the range from about 3.0 to about 3.5.

18. Use according to any one of claims 14 to 17, **characterized in that** the reaction medium is a buffered solution selected from the group consisting of sodium citrate/HCl, potassium hydrogen phthalate/HCl, citric acid/phosphate, and citrate-phosphate-borate/HCL.

19. Use according to any one of claims 14 to 18, **characterized in that** the concentration of the aminoacid in the reaction medium is within the range from about 10 mM to saturation.

20. Use according to any one of claims 14 to 19, **characterized in that** the concentration of the aminoacid in the reaction medium is within the range from about 25 mM to about 100 mM.

## Figure 1

1.03kx 20KV WD: 16mm 16/03/04 P: 03 Pericardio TQ-A ———— 10u

## Figure 3

LDH Test
MRC5-27°p. su pericardio bovino (72 h)

## Figure 2

Acc.V  Spot Magn    Det  WD ├─────────┤ 20 μm
20.0 kV 4.0   1000x    GSE 15.0   0.6 Torr pericardio 15h glutarald. A

## Figure 4

Neutral Red Test
MRC5-27°p. su pericardio bovino(72 h)

☐ Solo terreno
▨ Pericardio+cellule
▨ Polistirene+cellule

Absorbance (A540-A690)

# Figure 5

ALP Test
MRC5-27°p. su pericardio bovino (72 h)

Legend: Pericardio+cellule / Polistirene+cellule

# Figure 6

2.07kx  10KV  WD: 18mm  21/07/04  P: 04 Peric.72h          10u

European Patent
Office

**EUROPEAN SEARCH REPORT**

Application Number

EP 05 00 9748

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,Y | US 5 188 834 A (GRIMM ET AL) 23 February 1993 (1993-02-23) * the whole document * | 1-20 | A61L27/36 A61L31/14 |
| D,Y | US 5 873 812 A (CIANA ET AL) 23 February 1999 (1999-02-23) * the whole document * | 1-20 | |
| A | US 2004/005703 A1 (ABRAHAM GINGER A ET AL) 8 January 2004 (2004-01-08) * the whole document * | 1-20 | |
| A | WO 2005/009497 A (SCIMED LIFE SYSTEMS, INC; FREYMAN, TOBY; NAIMARK, WENDY; PALASIS, MARI) 3 February 2005 (2005-02-03) * the whole document * | 1-20 | |
| A | WO 02/40630 A (AMIEL, GILAD, E) 23 May 2002 (2002-05-23) * the whole document * | 1-20 | TECHNICAL FIELDS SEARCHED (IPC) |
| A | WO 2005/009225 A (COVALENT MEDICAL, INC; LOWINGER, JOHAN; LOWINGER, BRUNO; DELUSTRO, FRA) 3 February 2005 (2005-02-03) * the whole document * | 1-20 | A61L |

The present search report has been drawn up for all claims

| Place of search | Date of completion of the search | Examiner |
|---|---|---|
| The Hague | 9 November 2005 | Menidjel, R |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 1 719 533 A1**

## ANNEX TO THE EUROPEAN SEARCH REPORT
## ON EUROPEAN PATENT APPLICATION NO.

EP 05 00 9748

This annex lists the patent family members relating to the patent documents cited in the above-mentioned European search report.
The members are as contained in the European Patent Office EDP file on
The European Patent Office is in no way liable for these particulars which are merely given for the purpose of information.

09-11-2005

| Patent document cited in search report | | Publication date | Patent family member(s) | | Publication date |
|---|---|---|---|---|---|
| US 5188834 | A | 23-02-1993 | AT | 398276 B | 25-11-1994 |
| | | | AT | 132389 A | 15-03-1994 |
| | | | DE | 59008334 D1 | 09-03-1995 |
| | | | EP | 0401199 A2 | 05-12-1990 |
| | | | ES | 2067017 T3 | 16-03-1995 |
| US 5873812 | A | 23-02-1999 | DE | 69717519 D1 | 16-01-2003 |
| | | | DE | 69717519 T2 | 10-04-2003 |
| | | | EP | 0795337 A2 | 17-09-1997 |
| | | | ES | 2185827 T3 | 01-05-2003 |
| | | | IT | TO960178 A1 | 12-09-1997 |
| US 2004005703 | A1 | 08-01-2004 | NONE | | |
| WO 2005009497 | A | 03-02-2005 | US | 2005181016 A1 | 18-08-2005 |
| | | | US | 2005013870 A1 | 20-01-2005 |
| WO 0240630 | A | 23-05-2002 | AU | 2400302 A | 27-05-2002 |
| WO 2005009225 | A | 03-02-2005 | US | 2005069589 A1 | 31-03-2005 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5188834 A

- US 5873812 A

**Non-patent literature cited in the description**

- **GIRARDOT, M.N. et al.** Alpha-Aminoleic Acid, A New Compound,Prevents Calcification of Bioprostheticheart Valves. *The 17th Annual Meeting of the Society for Biomaterials,* 01 May 1991, 1141